(19) Europäisches Patentamt / European Patent Office / Office européen des brevets

(11) **EP 2 415 569 B1**

(12) **EUROPEAN PATENT SPECIFICATION**

(45) Date of publication and mention
of the grant of the patent:
**19.08.2020 Bulletin 2020/34**

(51) Int Cl.:
*B26F 1/38* *(2006.01)*        *A61F 13/15* *(2006.01)*
*A61F 13/49* *(2006.01)*        *A61F 13/494* *(2006.01)*

(21) Application number: **10758624.0**

(22) Date of filing: **29.03.2010**

(86) International application number:
**PCT/JP2010/055538**

(87) International publication number:
**WO 2010/113853 (07.10.2010 Gazette 2010/40)**

(54) **WORKING MACHINE AND METHOD OF WORKING SHEETS**

ARBEITSMASCHINE UND BAHNENBEARBEITUNGSVERFAHREN DAMIT

MACHINE DE TRAVAIL ET PROCÉDÉ DE TRAVAIL DE FEUILLES

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **03.04.2009 JP 2009091499**

(43) Date of publication of application:
**08.02.2012 Bulletin 2012/06**

(73) Proprietor: **Unicharm Corporation
Ehime 799-0111 (JP)**

(72) Inventors:
• **OONISHI, Hidetoshi
Kanonji-shi
Kagawa 769-1602 (JP)**
• **HAMADA, Akira
Kanonji-shi
Kagawa 769-1602 (JP)**
• **ISHIKAWA, Yasuyuki
Kanonji-shi
Kagawa 769-1602 (JP)**

(74) Representative: **Staeger & Sperling
Partnerschaftsgesellschaft mbB
Sonnenstraße 19
80331 München (DE)**

(56) References cited:
**DE-A1-102005 060 578        JP-A- 8 019 996
JP-A- H11 188 699        JP-A- 2002 052 496
JP-A- 2003 311 680        JP-A- 2009 000 776**

## Description

Technical Field

The invention relates to a processing apparatus which processing a sheet member an absorbent article used in manufacturing of and a method for processing the sheet member.

Background Art

Processing apparatuses that perform a processing such as cutting, joining to another member etc of a sheet member have already been known, the sheet member being used in manufacturing of an absorbent article. Some of such processing apparatuses include: a rotating roller which rotates with both ends thereof in an axial direction being supported; a sheet-placing portion having a sheet-placing surface on which the sheet member is placed; and a processing portion (for example, tools such as a blade, a pattern of joining etc) which is disposed on a peripheral surface of the rotating roller. And, those processing apparatuses perform some operations on the sheet member while the sheet member is being pinched between the sheet-placing surface and the processing portion (see patent literature 1, for example).

Citation List

[Patent Literature].

[PTL 1] Japanese Patent Application Laid-open Publication No. 11-188699 Further prior art in this technical field disclosing the preamble of claim 1 is represented by document DE 10 2005 060 578 A1.

Summary of the Invention

Technical Problem

In some cases, the foregoing processing portion provided on the peripheral surface of the rotating roller is asymmetry with respect to the axial direction of the rotating roller. That is, the processing portion is located closer to the one end with respect to the center in the axial direction of the rotating roller. In such a case, when the sheet member is pinched between the processing portion and the sheet-placing surface, moments are produced at parts which support both ends of the rotating roller in the axial direction, the moments being produced by the reaction forces which are exerted on the processing portion. The moments are different between the ends in the axial direction. Because of this difference between the moments, attitude of the rotating roller is inclined during the processing of the sheet member. This makes processing portion itself inclined. As a result, it is possible that, when the sheet member is pinched between the processing portion and the sheet-placing surface, the processing portion does not come into proper contact with the sheet member and the processing of the sheet member is not performed in good condition.

The invention has been made in view of the above problems, and an advantage thereof is to prevent the attitude of the rotating roller from being inclined and to perform the processing of the sheet member in good condition.

Solution to Problem

An aspect of the invention to solve the above problem is a processing apparatus for processing a sheet member used in manufacturing of an absorbent article according to claim 1, including:

a rotating roller that rotates with both ends thereof in an axial direction being supported;
a sheet-placing portion having a sheet-placing surface on which the sheet member is placed; and
a processing portion and a protuberance that are included on a peripheral surface of the rotating roller, wherein
the sheet member is processed by being pinched between the sheet-placing surface and the processing portion,
the processing portion is located at a side where one end of the rotating roller is positioned with respect to a center of the rotating roller in the axial direction of the rotating roller,
the protuberance is located at an other side where an other end of the rotating roller is positioned with respect to the center in the axial direction,
when the sheet member is pinched between the processing portion and the sheet-placing surface while the rotating roller is rotating, the protuberance abuts the sheet-placing surface.

Other features of this invention will become apparent from the description in this specification and the attached drawings.

Effects of the Invention

According to the invention, it is possible to prevent the attitude of the rotating roller from being inclined, and thereby to perform the processing of the sheet member in good condition.

BRIEF DESCRIPTION OF THE DRAWINGS

FIG. 1A is a side view of a diaper 1.
FIG. 1B is a rear view of the diaper 1.
FIG. 1C is a diagram showing the extended diaper 1.
FIG. 2 is a cross-sectional view of an absorbent main body 10 at the center in the longitudinal direction thereof.

FIG. 3 is a cross-sectional view of a solid gather section 19 of the diaper 1 while wearing the diaper 1.

FIG. 4 is a diagram showing a continuous body 30.

FIG. 5A is a diagram showing the diaper 1 in the course of production (Case 1).

FIG. 5B is a diagram showing the diaper 1 in the course of production (Case 2).

FIG. 5C is a diagram showing the diaper 1 in the course of production (Case 3).

FIG. 6 is a diagram showing how a die-cutting device 40 performs die-cutting.

FIG. 7 is a side view of the die-cutting device 40.

FIG. 8 is a cross-sectional view taken along line A-A in FIG. 7.

FIG. 9 is a developed figure of a peripheral surface 41a of a first rotating roller 41.

FIG. 10 is a diagram schematically showing the positional relationship of blades 46, 47, 48.

FIG. 11 is a cross-sectional view taken along line A-A in FIG. 7, showing the positions through which each of band base materials 20a, 22a passes.

FIG. 12 is a diagram of the comparative example, which is for describing effectiveness of the present embodiment.

FIGS. 13A to 13C are diagrams showing the modified example of a first peripheral surface 41a of the first rotating roller 41.

Mode for Carrying Out the Invention

At least the following matters will be made clear by the description in the present specification and the accompanying drawings.

Firstly, a processing apparatus for processing a sheet member used in manufacturing of an absorbent article, including: a rotating roller that rotates with both ends thereof in an axial direction being supported; a sheet-placing portion having a sheet-placing surface on which the sheet member is placed; and a processing portion and a protuberance that are included on a peripheral surface of the rotating roller, wherein the sheet member is processed by being pinched between the sheet-placing surface and the processing portion, the processing portion is located at a side where one end of the rotating roller is positioned with respect to a center of the rotating roller in the axial direction of the rotating roller, the protuberance is located at an other side where an other end of the rotating roller is positioned with respect to the center in the axial direction, when the sheet member is pinched between the processing portion and the sheet-placing surface while the rotating roller is rotating, the protuberance abuts the sheet-placing surface. In such a processing apparatus, it is possible to achieve the equilibrium of the moments produced at support positions of the both ends of the rotating roller in the axial direction, and to prevent attitude of the rotating roller from being inclined. Therefore, the processing of the sheet member can be performed in good condition.

Further, in such a processing apparatus described above and according to the invention, the processing apparatus further includes another protuberance that is disposed on the peripheral surface and is located closer to the side in the axial direction than the processing portion is to the side, while the sheet member is being pinched between the processing portion and the sheet-placing surface, the protuberance is in contact with the sheet-placing surface on the other side closer to the other end in the axial direction than the sheet member is to the other end, and the other protuberance abuts the sheet-placing surface on the side closer to the one end in the axial direction than the sheet member is to the one end, a distance from the protuberance to the center in the axial direction of the rotating roller is longer than a distance from the processing portion to the center in the axial direction and is longer than a distance from the other protuberance to the center in the axial direction. In such a configuration, the equilibrium of the moments produced at the support positions of the both ends of the rotating roller in the axial direction can be achieved, while avoiding the sheet member to get damaged by the contact with the protuberance.

Further, in such a processing apparatus described above, it is preferable that the processing apparatus further includes a first blade as the processing portion, a second blade as the protuberance, and a third blade as the other protuberance, and the sheet member is cut by being pinched between the sheet-placing surface and the first blade. In such a configuration, cutting failure caused by inclination of the attitude of the rotating roller can be prevented, and thereby, the cutting of the sheet member can be performed in good condition. Further, the equilibrium of the moments produced at the support positions of the both ends of the rotating roller in the axial direction can be achieved, while avoiding the sheet member to get cut (broken) by the contact with the second blade.

Further, in such a processing apparatus described above, it is preferable that each of the second blade and the third blade are included on the peripheral surface in a manner of stretching around the whole perimeter along a rotating direction of the rotating roller, a continuing direction of each of the second blade and the third blade is inclined at a certain angle with respect to the rotating direction. In such a configuration, durability of both second blade and third blade can be increased.

Further, in such a processing apparatus described above, it is preferable that the rotating roller is a first rotating roller that includes a first peripheral surface as the peripheral surface, the sheet-placing portion is a second rotating roller that includes a second peripheral surface as the sheet-placing surface and is supported rotatably, a motor that rotates each of the first rotating roller and the second rotating roller is provided individually on each of the rollers. In such a configuration, compared to the configuration in which the rotation is transmitted by a belt-pulley mechanism from one of the rotating rollers to the other rotating roller, both rotating rollers can rotate more smoothly. Therefore, the cutting of the sheet member can be performed in good condition.

Further, in such a processing apparatus described above, it is preferable that a circumferential velocity at which the first rotating roller rotates is different from a circumferential velocity at which the second rotating roller rotates. In such a configuration, an area which is in sheet-placing surface (that is, the second peripheral surface) and catches the first blade can change easily along the rotating direction of the second rotating roller. This makes it possible to keep a state so that the sheet member can be cut in good condition.

Further, preparing a rotating roller whose both ends in an axial direction are supported; and pinching the sheet member between a processing portion and a sheet-placing surface while the rotating roller is rotating and the sheet member is placed on the sheet-placing surface of the sheet-placing portion, the processing portion being included on a peripheral surface of the rotating roller and being located at a side where one end of the rotating roller is positioned with respect to a center of the rotating roller in the axial direction of the rotating roller, wherein when the sheet member is pinched between the processing portion and the sheet-placing surface while the rotating roller is rotating, a protuberance abuts the sheet-placing surface, the protuberance being included on the peripheral surface and being located closer to the other end to the center in the axial direction can be achieved. In such a method, it is possible to prevent attitude of the rotating roller from being inclined. Thereby, the processing of the sheet member can be performed in good condition.

=== Absorbent Article according to the Invention ===

In the present embodiment, providing a diaper 1 as an example of an absorbent article, a processing apparatus for processing a sheet member that is used for manufacturing the diaper 1 and a processing method will be described.

<< Configuration of Diaper 1 >>

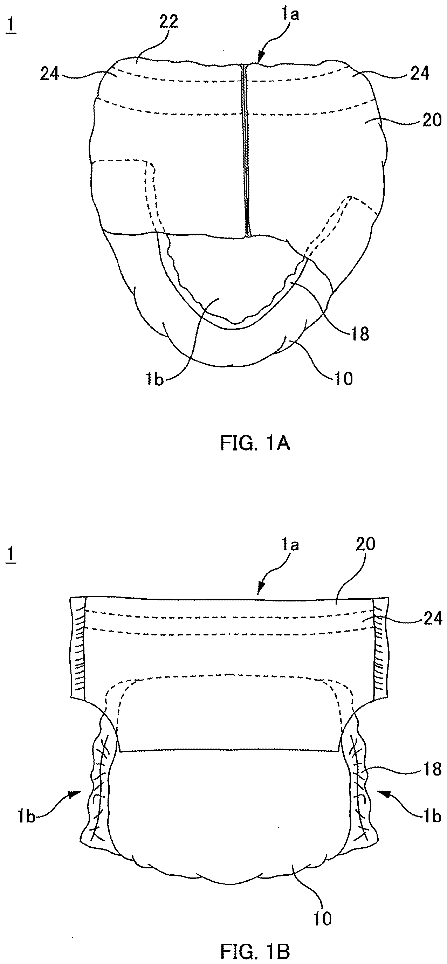

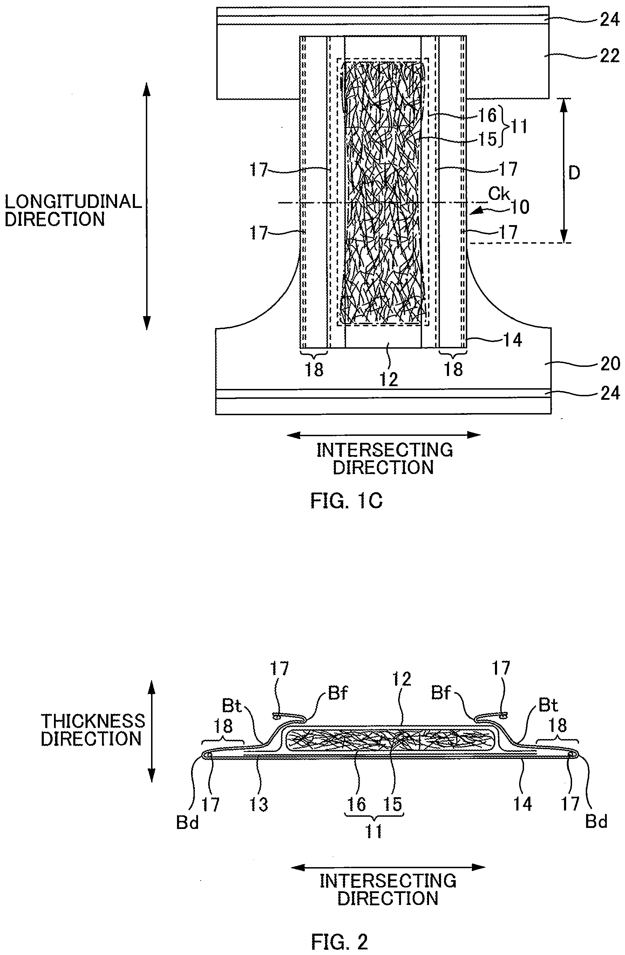

Firstly, the configuration of the diaper 1 will be described with reference to FIGS. 1A to 1C and 2. FIG. 1A is a side view of the diaper 1, and FIG. 1B is a rear view. FIG. 1C is a diagram of the extended diaper 1 viewed from a side which comes into contact with a wearer's skin. FIG. 2 is a cross-sectional view of an absorbent main body 10 at the center in the longitudinal direction thereof. In FIGS. 1C and 2, the following directions are indicated by arrows respectively: a longitudinal direction of the absorbent main body 10; a direction (hereinafter referred to as an intersecting direction) intersecting the longitudinal direction; and a thickness direction.

The diaper 1 includes: the absorbent main body 10 which comes into contact with the crotch of a wearer and absorbs bodily fluid such as urine; a back-side band 20 which covers a back-side part of the wearer; and a stomach-side band 22 which covers a stomach-side part of the wearer. In the extended form shown in FIG. 1C, the back-side band 20 and the stomach-side band 22 are lined up in parallel with a distance D therebetween; the absorbent main body 10 bridges them in such a manner as a contour is substantially H-shaped when viewed in a planar view. From that state, the diaper 1 is folded in two at a folding position Ck which is located at the center in the longitudinal direction of the absorbent main body 10. The bands 20, 22 which are opposite to each other with being two-folded are connected in an annular manner by attaching immovably at a portion which comes into contact with the sides of the wearer. This results in the diaper 1 which has a torso opening 1a and a pair of leg openings 1b formed thereon and is in the worn state (see FIGS. 1A and 1B). If an undetachable joined structure such as welding etc is used as the foregoing immovable attaching, the diaper 1 is a pull-on diaper product, and if a detachable joined structure such as a fastening tape member (not shown) etc is used, the diaper 1 is a wrap-style diaper product. In FIGS. 1A and 1B, the pull-on diaper product is provided as an example. Components of the diaper 1 will be described below.

As shown in FIG. 2, the absorbent main body 10 includes: an absorbent body 11; a surface sheet 12 (top sheet) which covers the absorbent body 11 from the skin-facing surface side (a surface of the side which touches the skin of the wearer); a back face sheet 13 (back sheet) which covers the absorbent body 11 from the opposite side to the surface sheet 12 (back face side); and an exterior sheet 14 (outer sheet) which is located on the back face side more outwardly than the back face sheet 13 and forms the exterior of the diaper 1. The absorbent body 11 is configured by an absorbent-body core 15 and a thin paper 16 such as tissue paper etc, the absorbent-body core 15 being formed by shaping liquid-absorbent fiber such as pulp fiber etc into a substantially guitar-shape when viewed from the top, the thin paper 16 wrapping the absorbent-body core 15. The absorbent-body core 15 may contain superabsorbent polymer (SAP). The surface sheet 12 is a fluid-permeable, nonwoven fabric sheet, and is larger than the absorbent body 11 in its planer size. The back face sheet 13 is a fluid-impermeable, film sheet, and is larger than the absorbent body 11 in its planer size. In a state of pinching the absorbent body 11 between the back face sheet 13 and surface sheet 12, the back face sheet 13 and surface sheet 12 are attached to each other in a frame-like manner at portions extending outwardly beyond four sides of the absorbent body 11.

The exterior sheet 14 is a nonwoven fabric sheet, and is larger than the back face sheet 13 and the surface sheet 12 in its planer size. In the exterior sheet 14, a portion extending outwardly in the intersecting direction which intersects the longitudinal direction of the absorbent main body 10 is folded back inwardly; the overlapping portions are joined in the vicinity of a folding-back position Bd. Further, in the vicinity of the folding-back position Bd, a stretchable member 17 such as rubber thread is fixed with stretching along the longitudinal direction of the absorbent main body 10. Therefore on both ends of the absorbent main body 10 in the intersecting direction, an around-leg gather section 18 is formed which realizes stretchability on the leg openings 1b of the diaper 1.

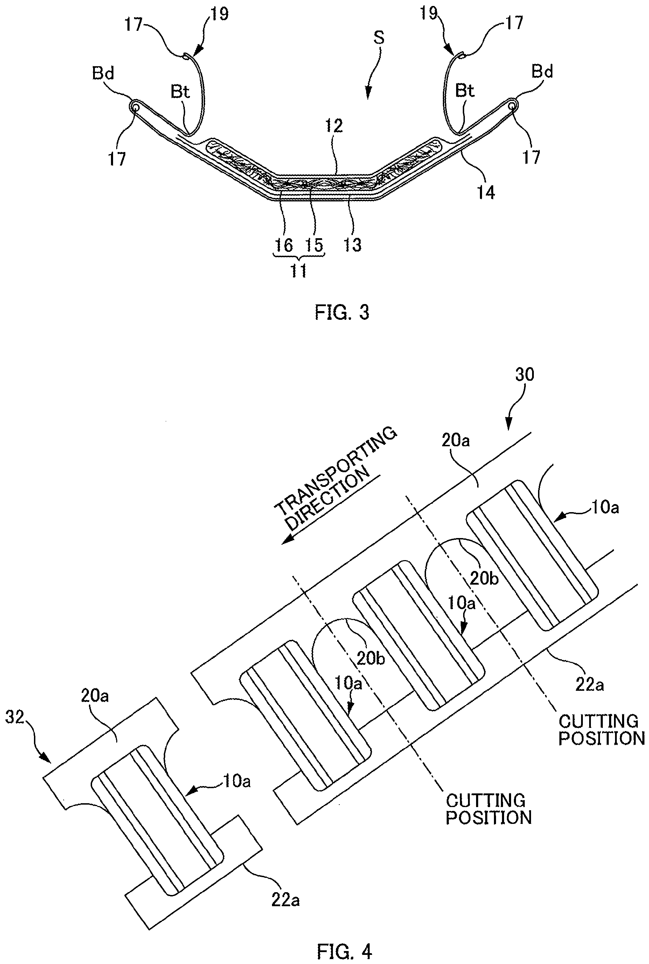

Further, the exterior sheet 14 which is folded back at the folding-back position Bd slightly raises at a raising position Bt located inwardly from the folding-back position Bd. Of the raising portions, portions which overlay the absorbent body 11 are folded back again outwardly in the intersecting direction (strictly speaking, the absorbent body 11 covers the surface sheet 12). In the vicinity of the end (free end) of the portions which are folded back at the folding-back position Bf, the stretchable member 17 is fixed with stretching along the longitudinal direction of the absorbent main body 10. Therefore in the absorbent main body 10, solid gather sections 19 are formed at positions where both ends of the absorbent body 11 in the intersecting direction (traversing direction) are located. As shown in FIG. 3, these solid gather sections 19 protrude such that the sections 19 fold up starting from the raising position Bt and bend backwards forming an overhang. And, the sections 19 abut around the groin of the wearer while the diaper 1 is in the worn state; thereby, the space S that catches excretion is formed between the solid gather sections 19. FIG. 3 is a cross-sectional view of the solid gather section 19 of the diaper 1 which is in the worn state.

The around-leg gather sections 18 and solid gather sections 19 are not limited to the configuration in which they are formed on the exterior sheet 14. It is preferable that the sections may be formed on a material other than the exterior sheet 14 (for example, other sheet member that is joined to skin-facing surface of the exterior sheet 14).

The back-side band 20 and stomach-side band 22 are thin band members made of flexible sheets such as nonwoven fabric etc, and both are cut in substantially rectangular shape when viewed from the top. The back-side band 20 and stomach-side band 22 intersect (are substantially perpendicular to) the longitudinal direction of the absorbent main body 10. The absorbent main body 10 is placed across the bands 20, 22 and its ends in the longitudinal direction are attached to and fixed on the central portion of each of the bands 20, 22 in the longitudinal direction. In the case of a configuration in which the bands 20, 22 consist of two sheets of nonwoven fabric, it is preferable that the ends of the absorbent main body 10 in the longitudinal direction are sandwiched between the sheets of the nonwoven fabric and are fixed. Further, it is also possible to give stretchability to the bands 20, 22 by fixing a stretchable member 24 such as rubber thread, rubber band etc to the bands 20, 22 while the stretchable member 24 stretching along the longitudinal direction of the bands 20, 22 (see FIG. 1C).

Further, in the present embodiment, the corners of the back-side band 20 (the corners on the side closer to the stomach-side band 22) are cut (more specifically, it is a process of cutting out; hereinafter referred to as the die-cutting process) in an arc shape inwardly from the end in the longitudinal direction to a position located outside the position where the end of the absorbent main body 10 in the longitudinal direction is attached. This improves the fittingness of the part forming the leg openings 1b in the back-side band 20 around the wearer's thigh. It is preferable that the die-cutting is performed not only for the back-side band 20, but also for the stomach-side band 22.

<< Manufacturing Method of Diaper 1>>

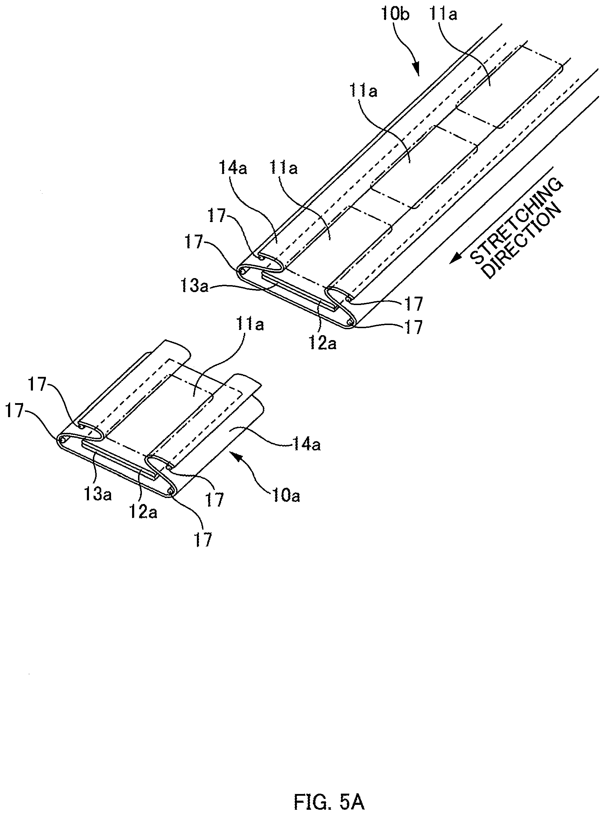

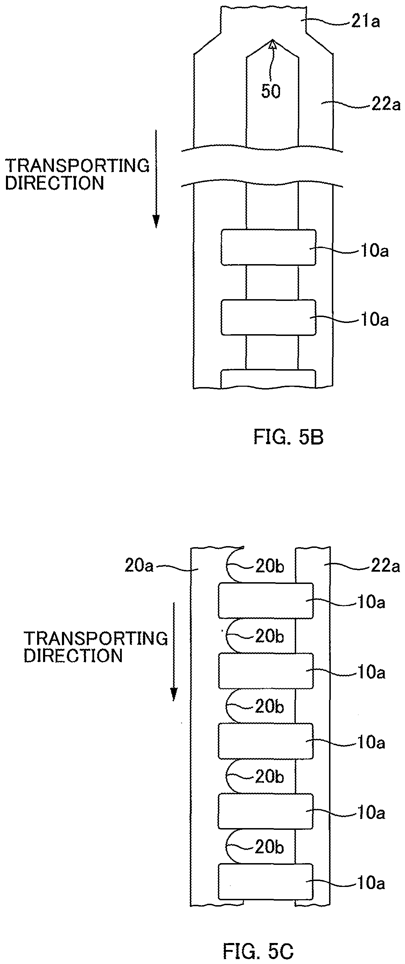

Next, the manufacturing method of the diaper 1 will be described with reference to FIGS. 4 and 5A to 5C. FIG. 4 is a diagram of a continuous body 30. FIGS. 5A to 5C are diagrams showing the diaper 1 in the course of production. For simplification of the figures, in FIGS. 5B and 5C, an absorbent-main-body base material 10a is drawn in a simplified form.

The diaper 1 is continuously produced on a continuous production line. On the continuous production line, the continuous body 30 shown in FIG. 4 is formed by joining the materials consisting of the diaper 1 while the materials is being conveyed in the transporting direction. The continuous body 30 is one in which a continuous-body piece 32 is stretching in the transporting direction, the continuous-body piece 32 forming the diaper 1 in the extended form. That is, when the continuous body 30 is cut into a product unit, the continuous-body piece 32 is formed. Thereafter, a final treatment is performed on the continuous-body piece 32 (for example, in the case of the pull-on diaper product, the continuous-body piece 32 is folded in two at the folding position Ck, and the bands 20, 22 are connected in an annular manner and are attached immovably), which results in finishing the diaper 1 as a product. The flow to forming of the continuous-body piece 32 will be described below.

In forming of the continuous body 30, firstly, a process in which the absorbent-main-body base material 10a is manufactured is performed, the absorbent-main-body base material 10a serving as a base material of the absorbent main body 10. The base material means a material on which the processing is performed and which finally becomes a component of the diaper 1; it is the same in the following. The absorbent-main-body base material 10a is manufactured by cutting a combined body 10b into a product unit, the combined body 10b being formed by combining base materials (the absorbent body 11, the surface sheet 12, the back face sheet 13, the exterior sheet 14, etc) of each component of the absorbent main body 10 (see FIG. 5A). The combined body 10b is continuous; inside the combined body 10b, absorbent-body base materials 11a (more specifically, the absorbent-body core 15 wrapped by the thin paper 16) are placed intermittently in a continuing direction of the combined body 10b with being sandwiched between the surface-sheet base material 12a and back-face-sheet base material 13a. Further, concerning an exterior-sheet base material 14a among the materials consisting of the combined body 10b, in order to form the around-leg gather section 18 or solid gather section 19 mentioned above, the stretchable member 17 is fixed to a certain portion of the material and is folded back at the folding-back position near the certain portion. When the combined body 10b is cut at a cutting position which is located between the absorbent-body base materials 11a in the continuing direction, the absorbent-main-body base material 10a is manufactured intermittently from an end section of the combined body 10b in the continuing direction, the absorbent-main-body base material 10a being substantially rectangular.

While the process of the abovementioned absorbent-main-body base material 10a is performed, a continuous back-side-band base material 20a and stomach-side-band base material 22a are conveyed in the transporting direction, which is along a continuing direction of the materials 20a, 22a. The band base materials 20a, 22a are each a material (raw fabric) as a base material of each of the back-side band 20 and stomach-side band 22; the band base materials 20a, 22a are examples of the sheet member used in manufacturing of the diaper 1. In the present embodiment, as shown in FIG. 5B, a slightly wide, undivided base material 21a is split in the width direction by a splitter 50 and divided into the back-side-band base material 20a and stomach-side-band base material 22a. The divided band base materials 20a, 22a are both conveyed in a the substantially parallel state lined up with a spacing in between the width of the spacing being the same as the spacing D between the bands 20, 22 in the finished product.

Further, while the band base materials 20a, 22a are each being conveyed along the continuing direction thereof as shown in FIG. 5B, a plurality of the absorbent-main-body base materials 10a, which are located at a regular interval along that continuing direction, are joined to each of the band base materials 20a, 22a. Concerning the joining method, any method appropriate to join the absorbent-main-body base material 10a and the band base materials 20a, 22a can be selected among the well-known joining methods. The absorbent-main-body base materials 10a are each placed across the band base materials 20a, 22a while the longitudinal direction of the base materials 10a is intersecting the continuing direction (that is, transporting direction) of the band base materials 20a, 22a. The plurality of absorbent-main-body base materials 10a are lined up along the continuing direction with a gap between the absorbent-main-body base materials 10a (see FIG. 5B).

After the absorbent-main-body base material 10a is joined to each of the band base materials 20a, 22a, each of the band base materials 20a, 22a continues to be conveyed, during which the die-cutting process is performed. The die-cutting process is a process for forming the leg openings 1b on the band base materials 20a, 22a; in the present embodiment, the process is performed only on the back-side-band base material 20a. In the die-cutting process, as shown in FIG. 5C, a portion of the back-side-band base material 20a is cut out in a substantially semi-circular shape, the portion being located between the absorbent-main-body base materials 10a in the continuing direction of the base material 20a. That is, the back-side-band base material 20a is die-cut so that a portion corresponding to the gap provided between an absorbent main bodies 10a is cut out.

In the one end (an end on the side opposite to the stomach-side-band base material 22a) of the back-side-band base material 20a in the width direction, substantially arc-shaped arches 20b are formed at a regular interval by the abovementioned die-cutting process. The die-cutting process will be described later in detail.

When the process mentioned above has ended, the continuous body 30 is formed. Thereafter, the continuous body 30 is cut at the cutting position located between the absorbent-main-body base materials 10a in a continuing direction of the continuous body 30 while the continuous body 30 is being conveyed in the continuing direction. Therefore, the continuous-body piece 32 is intermittently manufactured by the end section of the continuous body 30 in the continuing direction.

=== Die-Cutting Process ===

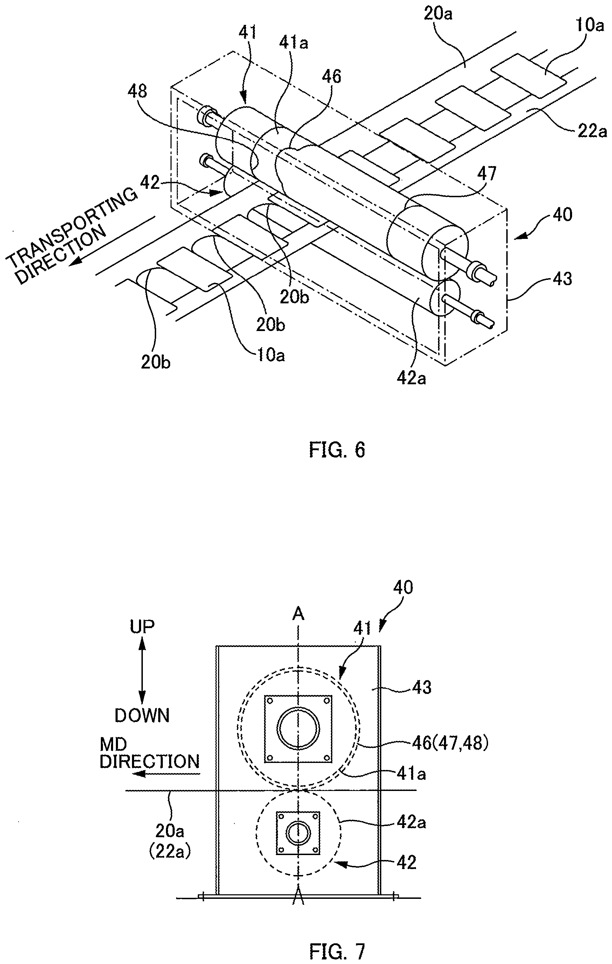

Next, the die-cutting process will be described in detail. The die-cutting process is performed by a die-cutting device 40 shown in FIG. 6. FIG. 6 is a diagram showing how the die-cutting device 40 performs the die-cutting. The die-cutting device 40 is an example of the processing apparatus according to the invention, and performs the die-cutting for the band base materials 20a, 22a, which serve as the sheet member used in manufacturing of the diaper 1. That is, a method of performing the die-cutting for the band base materials 20a, 22a with the die-cutting device 40 corresponds to a method for processing the band base materials 20a, 22a, and the die-cutting process corresponds to a physical operation process.

In the present embodiment, as mentioned above, the die-cutting is performed only on the back-side-band base material 20a (in other words, the stomach-side-band base material 22a is not cut and passes over inside the die-cutting device 40).

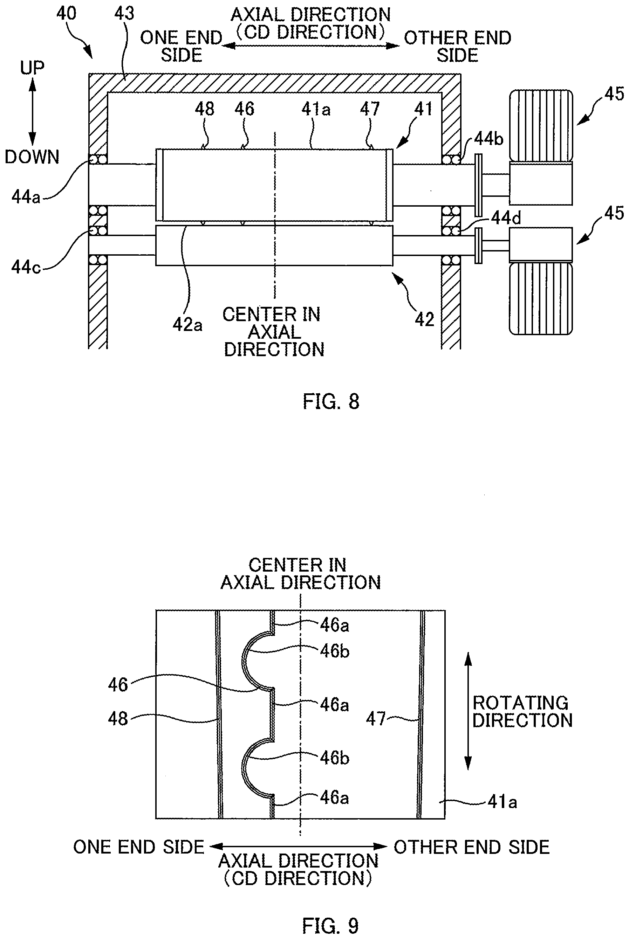

Below, the structure of the die-cutting device 40 will be described with reference to FIGS. 7 to 9. FIG. 7 is a side view of the die-cutting device 40. FIG. 8 is a cross-sectional view taken along line A-A in FIG. 7. FIG. 9 is a developed figure of a peripheral surface 41a of the first rotating roller 41. In the following description, a direction in which the band base materials 20a, 22a are conveyed is referred to as a MD direction, and a direction perpendicular to the MD direction is referred to as a CD direction. In other words, the MD direction corresponds to the continuing direction of the band base materials 20a, 22a, and the CD direction corresponds to the width direction of the band base materials 20a, 22a.

As shown in FIGS. 7 and 8, the die-cutting device 40 includes: a pair of upper and lower rotating rollers 41, 42, which rotates while their peripheral surfaces is being opposite to each other; a casing 43 which is substantially box-shaped and houses the pair of rotating rollers 41, 42; and motors 45 having a function as a driving source which is for rotating each of the pair of rotating rollers 41, 42.

Each of the pair of rotating rollers 41, 42 is supported at its both ends in the axial direction by side walls of the casing 43 via bearings 44a, 44b, 44c, 44d; in this state, the rollers 41, 42 rotates about an axis which is along the CD direction. The center of each of the rotating rollers 41, 42 in the axial direction is substantially the same as the middle position (in other words, the middle position between the bearings 44a, 44b and the middle position between the bearings 44c, 44d) between the support positions at which ends of the rotating rollers 41, 42 in the axial direction are supported, as shown in FIG. 8. Herein, the center of the rotating rollers 41, 42 in the axial direction is the center of the main parts of the rollers excluding the driving rods in the axial direction. By the die-cutting device 40, the band base materials 20a, 22a which are conveyed in the MD direction pass between the rotating rollers 41, 42, while this passage, the die-cutting is performed. That is, the band base materials 20a, 22a pass between the rotating rollers 41, 42 while the width direction is being along the axial direction of the rotating rollers 41, 42.

Further, the upper rotating roller 41, of the pair of rotating rollers 41, 42, is a cutting roller which includes a plurality of blades 46, 47, 48 on the peripheral surface 41a thereof; hereinafter referred to as the first rotating roller 41. The lower rotating roller 42 is an anvil roller on which the band base materials 20a, 22a are put over on its peripheral surface 42a and catches, on the peripheral surface 42a, the plurality of blades 46, 47, 48 included by the first rotating roller 41; hereinafter referred to as the second rotating roller 42.

That is, the die-cutting device 40 according to the present embodiment includes: the plurality of blades 46, 47, 48 which are included on the peripheral surface 41a of the first rotating roller 41 (hereinafter referred to as the first peripheral surface 41a); and the peripheral surface 42a of the second rotating roller 42 (hereinafter referred to as a second peripheral surface 42a). The second peripheral surface 42a corresponds to a sheet-placing surface on which the band base materials 20a, 22a are placed. In this regard, the second rotating roller 42 corresponds to a sheet-placing portion having the second peripheral surface 42a on which the band base materials 20a, 22a are placed in die-cutting process. The second rotating roller 42 is rotating with the band base materials 20a, 22a being placed on the second peripheral surface 42a. Thereby, the band base material 20a to be die-cut, that is the back-side-band base material 20a, moves towards a position where the die-cutting is performed (specifically, a position which is pinched between the first blade 46 and the second peripheral surface 42a to be described later). In addition, the back-side-band base material 20a on which the die-cutting is performed is transported downstream in the transporting direction from the foregoing die-cutting position. The second peripheral surface 42a is wider than the first peripheral surface 41a (see FIG. 8).

The plurality of blades 46, 47, 48 which are formed on the peripheral surface 41a of the first rotating roller 41 will be described more specifically. One of the plurality of blades 46, 47, 48 is a blade for the die-cutting, hereinafter referred to as the first blade 46. The first blade 46 corresponds to a processing portion, and is located at the side where the one end of the first rotating roller 41 is positioned with respect to the center in the axial direction of the first rotating roller 41. The first blade 46 is provided on the first peripheral surface 41a in a manner of stretching around the whole perimeter along the rotating direction (circumferential direction) of the first rotating roller 41 (see FIG. 9). Herein, the phrase "being located at the side where the one end is positioned in the axial direction" means "being located closer to the one end with respect to the center in axial direction of the first rotating roller 41".

Further, the first blade 46 includes: a linear part 46a extending straightly in the circumferential direction of the first rotating roller 41; and a curved part 46b which is curved in an arc-shaped manner in the circumferential direction. The back-side-band base material 20a is die-cut by this curved part 46b and the foregoing arch 20b is formed (see FIG. 6). That is, the die-cutting device 40 cuts (die-cuts) the back-side-band base material 20a by pinching between the second peripheral surface 42a and the first blade 46 (more specifically, the curved part 46b) while the band base materials 20a, 22a are passing between the rotating rollers 41, 42 with rotating both of the first rotating roller 41 and second rotating roller 42. Considering the stability of the first rotating roller 41 in attitude, it is preferable that a plurality of the curved parts 46b are arranged at a certain rotating angle along the rotating direction of the first rotating roller 41; more preferably, even number (two in the present embodiment) of the curved parts 46b are arranged.

The remaining blades 47 and 48 are blades which do not take part in die-cutting, and hereinafter respectively referred to as a second blade 47 and a third blade 48. The second blade 47 corresponds to a protuberance, and is located at the other side where the other end of the first rotating roller 41 is positioned with respect to the center in the axial direction of the first rotating roller 41 (see FIG. 9). Herein, the phrase "being located at the other side where the other end is positioned in the axial direction" means "being locating closer to the opposite end to the first blade 46, with respect to the center in axial direction of the first rotating roller 41". The third blade 48 corresponds to another protuberance, and is located closer to the one end in the axial direction than the first blade 46 is to the one end (see FIG. 9).

The second blade 47 and third blade 48 both protrude from the first peripheral surface 41a the same length as the first blade 46; the both blades are disposed on the first peripheral surface 41a around the whole perimeter along the rotating direction (circumferential direction) of the first rotating roller 41. That is, in the present embodiment, the second blade 47 and third blade 48 are located at positions where the first blade 46 is located in the rotating direction of the first rotating roller 41. Therefore, when the back-side-band base material 20a is pinched between the first blade 46 and the second peripheral surface 42a while the first rotating roller 41 is rotating, the second blade 47 and third blade 48 abut the second peripheral surface 42a.

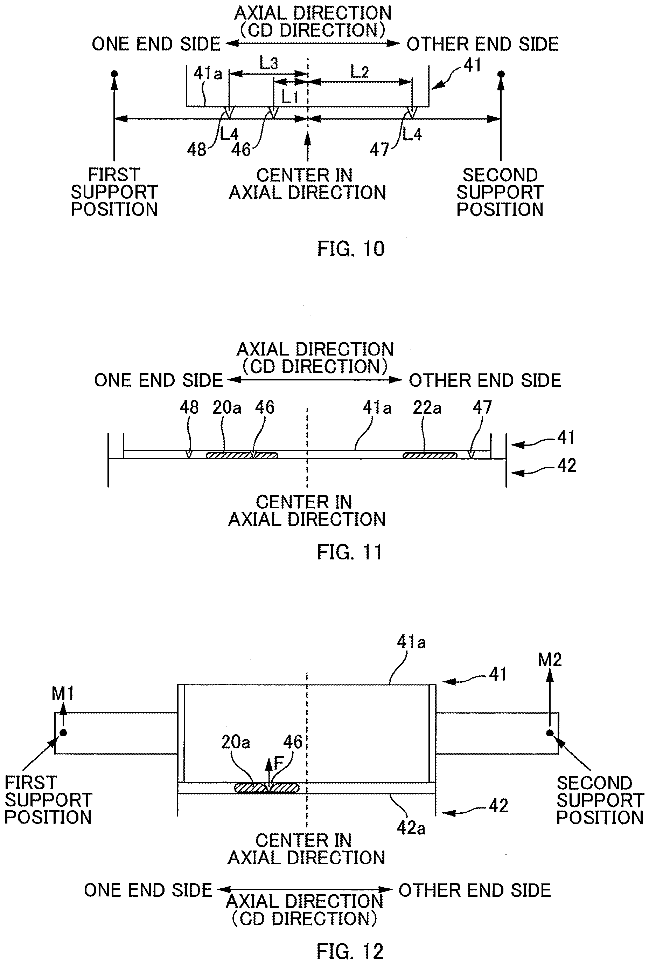

Further, a distance L2 from the second blade 47 (specifically, the center of gravity of the second blade 47 of in the axial direction; it is the same in the other blades 46, 48) in the axial direction of the first rotating roller 41 to the center of the first rotating roller 41 in the axial direction is longer than a distance L1 from the first blade 46 to the center in the axial direction. Also, the distance L2 is longer than a distance L3 from the third blade 48 to the center in the axial direction (see FIG. 10). This positional relationship will be described later in detail. It should be noted that the continuing direction of each of the second blade 47 and third blade 48 is inclined at a certain angle (preferably approximately 1°) with respect to the rotating direction of the first rotating roller 41. This makes it possible to prevent the second blade 47 or the third blade 48 from bending (slanting such that the direction of protruding gets inclined radially of the first rotating roller) comparing to the case in which the continuing direction of each of the second blade 47 and third blade 48 is the same as the rotating direction of the first rotating roller 41. As a result, the durability of each of the second blade 47 and third blade 48 increases.

The motor 45 is a so-called servomotor, and is connected with a coupling at the end of the part protruding the casing 43, in the rotational axis of the rotating rollers 41, 42, as shown in FIG. 8. In the present embodiment, as shown in the figure, the motors 45 are provided individually on each of the first rotating roller 41 and second rotating roller 42. This enables both rotating rollers 41, 42 to rotate more smoothly; therefore, the die-cutting can be performed in good condition.

Specifically speaking, in the case of configuration in which belt-pulley mechanism transmits rotation of either one of the rotating rollers 41, 42 to the other of the rotating rollers 41, 42, it is possible to produce looseness due to roughness of a driving belt, to prevent, due to the looseness, a proper rotation of the rotating roller to which the rotation is transmitted, and to prevent die-cutting from being performed normally. As opposed thereto, in the present embodiment, the motors 45 are provided individually on each of the first rotating roller 41 and second rotating roller 42. Therefore, looseness is not produced, and both rotating rollers 41, 42 smoothly rotate. As a result, the die-cutting can be performed in good condition.

Further, in the present embodiment, the power output of each of the motors 45 is adjusted so that the circumferential velocity at which the first rotating roller 41 rotates is different from the circumferential velocity at which the second rotating roller 42 rotates. This makes it possible to change easily, along the rotating direction of the second rotating roller 42, the area which is in the second peripheral surface 42a and catches the first blade 46 (the area which pinches the back-side-band base material 20a together with the first blade 46). This makes it possible to keep a state so that the die-cutting can be performed in good condition.

Specifically speaking, if the first rotating roller 41 and the second rotating roller 42 rotates at the same circumferential velocity, the area which is in the second peripheral surface 42a and catches the first blade 46 gets limited to a certain area. As a result, the localized area has worn, and target materials (specifically, the back-side-band base material 20a) for die-cutting cannot be pinched normally between the second peripheral surface 42a and first blade 46. Therefore, the die-cutting is not performed in good condition. As opposed thereto, in the present embodiment, the circumferential velocity at which first rotating roller 41 rotates is different from the circumferential velocity at which the second rotating roller 42 rotates. Therefore, the area which is in the second peripheral surface 42a and catches the first blade 46 changes, which results in avoiding the localized wear. As a result, the foregoing target materials can be pinched properly between the second peripheral surface 42a and the first blade 46, the die-cutting can be performed steadily in good condition.

Further, in the present embodiment, the first rotating roller 41 and second rotating roller 42 are different in external diameter (more specifically, the external diameter of the main part of the roller) from each other. Specifically, the external diameter of the first rotating roller 41 is slightly larger than the external diameter of the second rotating roller 42 (see FIGS. 6 to 8). If, as mentioned above, the rotating rollers 41, 42 are different in external diameter from each other, the area which is in the second peripheral surface 42a and catches the first blade 46 is more likely to change. As a result, an effect that the foregoing localized wear is avoid becomes remarkably. However, the invention is not limited thereto, each of rotating rollers 41, 42 may be substantially same in external diameter.

<< Concerning Positional Relationship of Blades 46, 47, 48 >>

Next, the positional relationship of the blades 46, 47, 48 which are included on the first peripheral surface 41a of the first rotating roller 41 will be described with reference to FIG. 10. FIG. 10 is a diagram schematically showing the positional relationship of the blades 46, 47, 48. In the following description, a position where a one end section in the axial direction of the first rotating roller 41 is supported (in other words, a position of the bearings 44a closer to the one end in the CD direction) is referred to as a first support position, and a position where another end section in the axial direction of the first rotating roller 41 is supported (in other words, a position of the bearings 44b closer to the other end in the CD direction) is referred to as a second support position.

In the present embodiment, as shown in FIG. 10, the first support position and second support position respectively is L4 away from the center in the axial direction of the first rotating roller 41. On the other hand, as mentioned above, a distance L2 from the second blade 47 to the center in the axial direction is longer than a distance L1 from the first blade 46 to the center in the axial direction, and is longer than a distance L3 from the third blade 48 to the center in the axial direction. Further, concerning the distance L3 from the third blade 48 to the center in the axial direction, the distance is set to fulfill the following relationship.

$$L3 = L2 - L1$$

The purpose of setting the placements of the blades 46, 47, 48 as mentioned above is to achieve the equilibrium of the moments between the support positions, the moment being produced from the first support position and second support position as the center points by the reaction forces that are exerted on each of the blades 46, 47, 48. The detail thereof will be described later.

<< Action of Die-Cutting Device 40 >>

Next, concerning the action of the die-cutting device 40, steps of the die-cutting process performed by the die-cutting device 40 is will be described.

The back-side-band base material 20a and stomach-side-band base material 22a are conveyed along the MD direction, and then are inserted into the die-cutting device 40. These base materials 20a, 22a which are inserted into the die-cutting device 40 with the base materials 20a, 22a being put over the peripheral surface 42a (that is, the second peripheral surface 42a) of the second rotating roller 42 which is rotating. The base materials 20a, 22a pass between the first rotating roller 41 and second rotating roller 42. Meanwhile, the first rotating roller 41 rotates at a different circumferential velocity from the second rotating roller 42. The blades 46, 47, 48 rotates while the blades are being opposite to the second peripheral surface 42a, the blades 46, 47, 48 being disposed on the peripheral surface 41a (that is, the first peripheral surface 41a) of the first rotating roller 41. At this time, a certain portion (a portion which is cut out in order to form the leg openings 1b) closer to the one end of the back-side-band base material 20a in the CD direction is pinched between the first blade 46 (more specifically, a curved part 46b) and the second peripheral surface 42a. In other words, the back-side-band base material 20a, of the band base materials 20a, 22a, passes between the rotating rollers 41, 42 so that the material 20a comes into contact with the first blade 46, as shown in FIG. 11. FIG. 11 is a diagram showing the positions through which the band base materials 20a, 22a pass in the cross-section taken along line A-A in FIG. 7.

The die-cutting device 40 performs the die-cutting on a certain portion which is located closer to the one end of the back-side-band base material 20a in the CD direction, by pinching the certain portion between the curved part 46b of the first blade 46 and the second peripheral surface 42a. As a result, a portion, of the back-side-band base material 20a, which has passed a position at which the base material 20a is pinched between the first blade 46 and the second peripheral surface 42a in the MD direction, is in the state shown in FIG. 5B. That is, the state in which a certain portion closer to the one end in the CD direction is cut out into a semi-circular shape and the arch 20b are formed at a regular interval.

As mentioned above, in the die-cutting process, the back-side-band base material 20a is die-cut by being pinched between the first blade 46 provided on the first peripheral surface 41a of the first rotating roller 41 and the second peripheral surface 42a of the second rotating roller 42, while the first rotating roller 41 and second rotating roller 42 are rotating and the band base materials 20a, 22a are placed on the second peripheral surface 42a.

Herein, as mentioned above, in order to bring the first blade 46 into contact with the back-side-band base material 20a of the band base materials 20a, 22a conveyed while being lined up in the CD direction, the first blade 46 (more specifically, curved part 46b) is arranged at a position which is slightly closer to the one end with respect to the center of the first rotating roller 41 in the axial direction. That is, the foregoing distance L1 is the distance which is adjusted so that a to-be-die-cut portion of the back-side-band base material 20a is pinched by the first blade 46 together with the second peripheral surface 42a. In other words, the back-side-band base material 20a is conveyed with being located closer to one end in the CD direction than the stomach-side-band base material 22a is to one end. In addition, the back-side-band base material 20a passes the range which is within the gap between the rotating rollers 41, 42 and in which the base material 20a comes into contact with the first blade 46 in the CD direction (in other words, the axial direction of the rotating rollers 41, 42) (see FIG. 11).

On the other hand, the stomach-side-band base material 22a is conveyed with being located closer to the other end in the CD direction than the back-side-band base material 20a is to the other end. In addition, the stomach-side-band base material 22a passes the range which is within the gap between the rotating rollers 41, 42 and in which the stomach-side-band base material 22a does not come into contact with any of the blades 46, 47, 48 in the CD direction (see FIG. 11). More specifically, the stomach-side-band base material 22a passes between the rotating rollers 41, 42 while the stomach-side-band base material 22a is being located at the other side where the other end of the first rotating roller 41 is positioned with respect to the center in the axial direction of the first rotating roller 41, and is being located closer to the one end than the second blade 47 is to the one end. In other words, the second blade 47 and third blade 48 are placed at the positions where the blades do not come into contact with the band base materials 20a, 22a in the axial direction of the first rotating roller 41. That is, the foregoing distances L2 and L3 are the distances which are adjusted so that the second blade 47 and third blade 48 do not come into contact with the band base materials 20a, 22a and abut only the second peripheral surface 42a. Therefore, while the back-side-band base material 20a is being pinched between the first blade 46 and second peripheral surface 42a, the second blade 47 abuts the second peripheral surface 42a on the side closer to the other end in the CD direction (the side closer to the other end in the axial direction) than the back-side-band base material 20a and stomach-side-band base material 22a are to the other end. Also, the third blade 48 abuts the second peripheral surface 42a on the side closer to the one end in the CD direction (the side closer to the one end in the axial direction) than the back-side-band base material 20a and stomach-side-band base material 22a are to the one end.

<< Flow prior to Die-Cutting Process until Die-Cutting Process >>

In the stage prior to inserting the band base materials 20a, 22a into the die-cutting device 40 (that is, prior to the die-cutting process), the absorbent-main-body base material 10a is joined to each of the band base materials 20a, 22a; further, the stretchable member 24 is joined thereto, the stretchable member 24 being for realizing stretchability on each of the band base materials 20a, 22a.

More specifically, a plurality of the absorbent-main-body base materials 10a each are joined to the band base materials 20a, 22a with being lined up along the continuing direction (the transporting direction of the band base materials 20a, 22a) of the band base materials 20a, 22a (see FIG. 5B). Further, although not shown in the figure, in a stretching state along the foregoing transporting direction, the stretchable member 24 is joined to an outer end section of each of the band base materials 20a, 22a in the width direction. In this state, each of the band base materials 20a, 22a is put over on peripheral surface of the second rotating roller 42. When the band base materials 20a, 22a pass between rotating rollers 41, 42, the die-cutting process is performed on the back-side-band base material 20a. This results in cutting out of a portion of the back-side-band base material 20a in a semi-circular shape, the portion being located between the absorbent-main-body base materials 10a in the continuing direction. Therefore, in the present embodiment, the die-cutting process is performed while the absorbent-main-body base material 10a and stretchable member 24 are joined to each of the band base materials 20a, 22a.

In other words, the die-cutting device 40 is a device for cutting a continuous sheet (specifically speaking, the back-side-band base material 20a) that is used for manufacturing the diaper 1. The die-cutting device 40 includes the pair of rotating rollers 41, 42 which rotates while their peripheral surfaces are opposite to each other. The continuous sheet includes: the stretchable member 24 stretching along a continuing direction of the continuous sheet; and a plurality of the absorbent-main-body base materials 10a lined up in the continuing direction. One of the rotating rollers (specifically, the second rotating roller 42) rotates while the continuous sheet is putting over the peripheral surface thereof, the continuous sheet including the plurality of absorbent-main-body base materials 10a and the stretchable member 24. The other of the rotating rollers (specifically, the first rotating roller 41) has, on its peripheral surface, a blade (specifically, the first blade 46) for cutting the continuous sheet. When the continuous sheet passes between a pair of the rotating rollers 41, 42, the continuous sheet is cut with the foregoing blade so that a portion of the continuous sheet is cut out, the portion being located between the absorbent-main-body base materials 10a in the continuing direction.

In the abovementioned workflow, in the present embodiment, it becomes possible to perform proper die-cutting when the absorbent main body 10a has been joined to each of the band base materials 20a, 22a properly.

Specifically speaking, if each of the band base materials 20a, 22a is inserted into the die-cutting device 40 while only the stretchable member 24 is joined (that is, the absorbent main body 10a is not joined), each of the band base materials 20a, 22a firstly is put over the peripheral surface 42a of the second rotating roller 42. At this time, tension is produced substantially uniformly throughout the band base materials 20a, 22a. As a result, each of the band base materials 20a, 22a overcomes the repelling force from the stretchable member 24 and gets adequately stretched in the continuing direction.

However, if the back-side-band base material 20a is die-cut under the foregoing condition so that the material is cut out at a regular interval along its continuing direction, a portion adjacent to the die-cut portion in the continuing direction (that is, a portion located between arch 20b adjacent thereto, hereinafter referred to as an adjacent portion) will become a free end. Therefore, the foregoing tension does not act on the adjacent portion. This results in twisting of the adjacent portion, or shrinkage along the continuing direction caused by the the repelling force from the stretchable member 24. Thereafter, the absorbent-main-body base material 10a is joined to an adjacent portion. However, it is possible that the absorbent-main-body base material 10a is not joined properly in the foregoing state.

As opposed thereto, in the present embodiment, the absorbent-main-body base material 10a is joined to the band base materials 20a, 22a prior to the die-cutting process. Further, the die-cutting is performed on the portion, of the back-side-band base material 20a, that is located between the absorbent-main-body base materials 10a in the continuing direction. In other words, the absorbent-main-body base material 10a is joined in advance to a corresponding portion to an adjacent portion. As a result, concerning the foregoing portion correspond to the adjacent portion, rigidity increases; also the portion becomes less likely to be twisted due to joining the absorbent-main-body base material 10a. In addition, the shrinkage caused by the the repelling force from the stretchable member 24 can be restricted by the absorbent-main-body base material 10a. Therefore, in the present embodiment, the die-cutting can be performed normally while the absorbent main body 10a is being properly joined to the band base materials 20a, 22a.

=== Effectiveness of the Present Embodiment ===

In the die-cutting device 40 and die-cutting process according to the present embodiment, it is possible to prevent the attitude of the first rotating roller 41 from being inclined. Also, the die-cutting of the band base materials 20a, 22a (back-side-band base material 20a in the present embodiment) can be performed in good condition. The effectiveness of the present embodiment will be described below in detail.

As described in the section of Technical Problem, in some cases, a blade (first blade 46) for the die-cutting provided on the peripheral surface 41a of the first rotating roller 41 (the first peripheral surface 41a) is asymmetry with respect to the axial direction of the first rotating roller 41 and is located at the side where the one end of the first rotating roller 41 is positioned with respect to the center in the axial direction of the first rotating roller 41. In such a case, as shown in FIG. 12, moments M1 and M2 are different between the support positions, the moments M1 and M2 being produced respectively at the first support position and second support position by the reaction force F which is exerted on the first blade 46a when the band base material 20a is pinched between the first blade 46 and the peripheral surface 42a of the second rotating roller 42 (the second peripheral surface 42a). Specifically speaking, the produced moment M2 is greater at the support position farther from the first blade 46, that is, the second support position. FIG. 12 is a diagram of the comparative example, which is for describing effectiveness of the present embodiment and shows the magnitude of the moments M1 and M2 which are produced at the first support position and second support position.

The difference between the abovementioned moments M1 and M2 causes difference between the forces which exert respectively on the support positions (forces which press down the bearings 44a, 44b). Due to the difference between those forces, attitude of the first rotating roller 41 is inclined during die-cutting (specifically speaking, the axial direction, which is supposed to be parallel to the CD direction, of the first rotating roller 41 is inclined with respect to the CD direction). As a result, the first blade 46 itself gets inclined. As a result, when the band base material 20a is pinched between the first blade 46 and the second peripheral surface 42a, the band base material 20a cannot abut properly; therefore, it is possible that the die-cutting of the band base material 20a is not performed in good condition. Especially, in the case of a thin fibrous sheet made of nonwoven fabric etc such as the band base materials 20a, 22a, even if the blade 46 is inclined slightly, some of fibers are left uncut in a section which is supposed to cut and the die-cutting cannot be performed in good condition. If, as mentioned above, the die-cutting is performed on a thin fibrous sheet as a target, the abovementioned problem that the die-cutting becomes difficult due to inclination of the attitude of the first rotating roller 41 becomes more prominent.

As opposed thereto, in the present embodiment, on the first peripheral surface 41a of the first rotating roller 41, the second blade 47 is included which is located at the other side where the other end is positioned with respect to the center in the axial direction of the first rotating roller 41. In addition, when the band base material 20a is pinched between the first blade 46 and the second peripheral surface 42a while the first rotating roller 41 is rotating, the second blade 47 abuts the second peripheral surface 42a. This makes it possible to achieve the foregoing equilibrium of the moments M1 and M2.

That is, the second blade 47 is included on the opposite side to the side on which the first blade 46 is included with respect to the center in axial direction of the first rotating roller 41. Consider that the second blade 47 abuts the second peripheral surface 42a while the band base material 20a is being pinched between the first blade 46 and the second peripheral surface 42a. While the reaction force F is exerting on the first blade 46, a reaction force which is substantially equal in magnitude is also exerting on the second blade 47. Concerning the moments M1 and M2 which are produced at the first support position and second support position (strictly speaking, the resultant moment which is the sum of the moment produced by the reaction force exerted on the first blade 46 and the moment produced by the reaction force exerted on the second blade 47), the difference between the moments M1 and M2 can decrease. This prevents the attitude of the first rotating roller 41 from getting inclined during die-cutting; thereby, the die-cutting can be performed in good condition. That effect is especially effective in die-cutting which targets a thin fibrous sheet, and the present embodiment is a preferable embodiment for continuously producing the diaper 1 having the foregoing shape.

Further, in the present embodiment, on the first peripheral surface 42a, the third blade 48 is also included which is located closer to the one end in the axial direction than the first blade 46 is to the one end. In addition, the distance L2 from the second blade 47 to the center of the first rotating roller 42 in axial direction is longer than the distance L1 from the first blade 46 to the center in the axial direction, and is longer than the distance L3 from the third blade 48 to the center in the axial direction. While the back-side-band base material 20a is being pinched between the first blade 46 and the second peripheral surface 42a, the second blade 47 abuts the second peripheral surface 42a on the side closer to the other end in the axial direction than the back-side-band base material 20a and stomach-side-band base material 22a are to the other end. Also, the third blade 48 abuts the second peripheral surface 42a on the side closer to the one end in the axial direction than the back-side-band base material 20a and stomach-side-band base material 22a are to the one end. Therefore, the equilibrium of the moments M1 and M2 can be achieved while avoiding the band base materials 20a, 22a to get cut (broken) by the contact with the second blade 47.

Specifically speaking, in order to achieve the equilibrium of the moments M1 and M2, it is ideal that the second blade 47 is located at a symmetric position with respect to the first blade 46 about the center of the first rotating roller 42 in the axial direction (in other words, the distance L2 from the second blade 47 to the center in the axial direction is equal to the distance L1 from the first blade 46 to the center in the axial direction). However, the second blade 47 cannot be placed at the ideal position stated above under the condition that only the back-side-band base material 20a is die-cut and the condition that the second blade 47 does not bring into contact with the band base materials 20a, 22a. Therefore, in the present embodiment, the second blade 47 is included at a position located slightly closer to the one end in the axial direction with respect to the foregoing ideal position. However, therefore, it is impossible to solve sufficiently the difference between the moments M1 and M2. Therefore, the equilibrium of the moments M1 and M2 can be achieved by including the third blade 48 which is located closer to the one end in the axial direction than the first blade 46 is to the one end, and by setting the placement of the third blade 48 in the position which fulfills the foregoing requirement (L3 = L2 - L1) in the axial direction of the first rotating roller 42.

=== Other Embodiments ===

In the foregoing embodiment, the die-cutting device 40 and the method for die-cutting according to the invention are mainly described. However, the foregoing embodiments are for the purpose of elucidating the understanding of the invention, and are not construed as limiting the invention in any way. The invention can of course be altered and improved without departing from the gist thereof, and equivalents are intended to be embraced therein. Further, the foregoing settings, sizes, and shapes etc are examples only to demonstrate the effects of the invention, and are not construed as limiting the invention in any way.

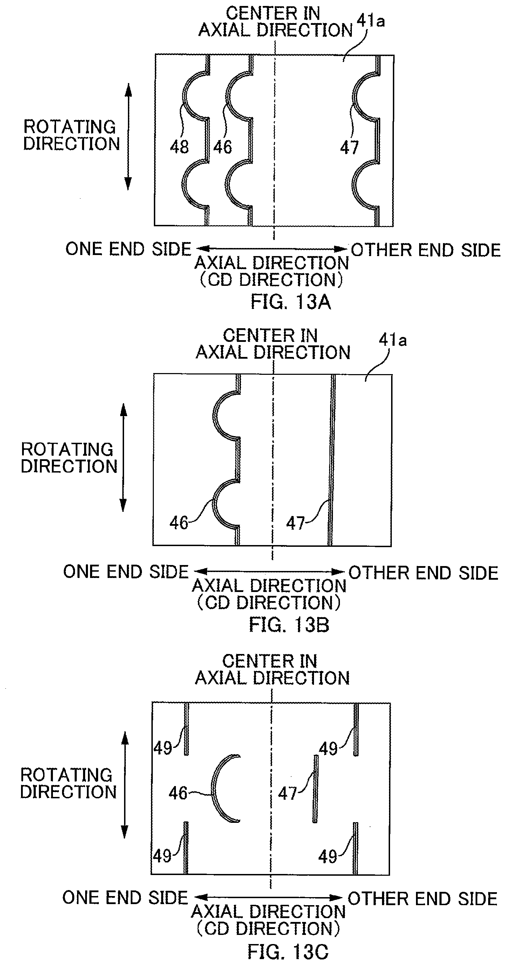

Especially, the shapes and placements of the blades 46, 47, 48 are not limited to the foregoing embodiment. For example, embodiments shown in FIGS. 13A to 13C can be employed. FIGS. 13A to 13C are diagrams showing of the first peripheral surface 41a of the first rotating roller 41 of the modified examples, and show the first peripheral surface 41a in the extended form. The embodiment shown in FIG. 13A is one that the shapes of the second blade 47 and third blade 48 are the same as that of the first blade 46. The embodiment shown in FIG. 13B is one that only the first blade 46 and second blade 47 are included (that is, an embodiment that does not included the third blade 48). The embodiment shown in FIG. 13C is one that only the first blade 46 and second blade 47 are included and the blades 46, 47 are not continuous in the rotating direction of the first rotating roller 41. In the embodiment shown in FIG. 13C, a so-called dummy blade 49 is included in a position where the first blade 46 and second blade 47 do not exist in the rotating direction of the first rotating roller 41, and reduces the stress concentration the first blade 46 or the second blade 47.

Further, in the foregoing embodiment, the second rotating roller 42 corresponds to a sheet-placing portion on which the sheet member to be processed (the band base materials 20a, 22a; the back-side-band base material 20a in the foregoing embodiment) is placed. Also, its peripheral surface 42a (the second peripheral surface 42a) is a sheet-placing surface. However, the invention is not limited thereto. For example, It is preferable that a sheet-placing table on which the sheet member is placed is included as a sheet-placing portion and that the upper surface of the sheet-placing table is the sheet-placing surface.

Further, in the foregoing embodiment, the description is made providing the apparatus (die-cutting device 40) and method for die-cutting the sheet member to be processed as an example. That is, in the foregoing embodiment, the die-cutting process in which the die-cutting is performed using a blade (first blade 46) as a processing portion is described as an example of physical operation process. However, the invention is not limited thereto. As long as the process is one that is performed with the sheet member being pinched between the processing portion and the sheet-placing surface, it is preferable to be other physical operation processes (for example, a compressing-embossing process in which embosses are formed on the sheet member, or a joining process in which the sheet members are joined can be employed). That is, the processing portion is not limited to the blade and can be any other parts for processing. In the same way, the protuberance and the other protuberance are not limited to the blades (the second blade 47 and the third blade 48); a protrusion or projection may be employed.

Further, in the foregoing embodiment, the processing of the sheet member (that is, the band base materials 20a, 22a) which is used in manufacturing of the diaper 1 is described providing the diaper 1 as an example of an absorbent article. However, the invention is not limited thereto. For example, a sanitary napkin, incontinence pad, wiper etc can be provided as other example of the absorbent articles, and the invention can be applied for processing the sheet member used in manufacturing of these products.

Reference Signs List

1 diaper (absorbent article), 1a torso opening, 1b leg opening, 10 absorbent main body, 10a absorbent-main-body base material, 10b combined body, 11 absorbent body, 11a absorbent-body base material, 12 surface sheet, 12a surface-sheet base material, 13 back face sheet, 13a back-face-sheet base material, 14 exterior sheet, 14a exterior-sheet base material, 15 absorbent-body core, 16 thin paper, 17 stretchable member, 18 around-leg gather section, 19 solid gather section, 20 back-side band, 20a back-side-band base material (sheet member), 20b arch, 21a undivided base material, 22 stomach-side band, 22a stomach-side-band base material (sheet member), 24 stretchable member, 30 continuous body, 32 continuous-body piece, 40 die-cutting device (processing apparatus), 41 first rotating roller, 41a first peripheral surface (peripheral surface), 42 second rotating roller (sheet-placing portion), 42a second peripheral surface (sheet-placing surface), 43 casing, 44a, 44b, 44c, 44d bearings, 45 motor, 46 first blade (blade, processing portion), 46a linear part, 46b curved part, 47 second blade (protuberance), 48 third blade (another protuberance), 49 dummy blade, 50 splitter

**Claims**

1. A processing apparatus for processing a sheet member used in manufacturing of an absorbent article, comprising:

   a rotating roller (41) that rotates with both ends thereof in an axial direction being supported;
   a sheet-placing portion (42) having a sheet-placing surface (42a) on which the sheet member is placed; and
a processing portion (46) and a protuberance (47) that are included on a peripheral surface of the rotating roller, wherein
the sheet member is processed by being pinched between the sheet-placing surface (42) and the processing portion (46),
the processing portion (46) is located at a side where one end of the rotating roller (41) is positioned with respect to a center of the rotating roller in the axial direction of the rotating roller,
the protuberance (47) is located at an other side where an other end of the rotating roller is positioned with respect to the center in the axial direction,
the protuberance (47) abuts the sheet-placing surface (42a) when the sheet member is pinched between the processing portion (46) and the sheet-placing surface while the rotating roller (41) is rotating, **characterized in that**
the processing apparatus further comprises another protuberance (48) that is disposed on the peripheral surface and is located closer to the side in the axial direction than the processing portion (46) is to the side,
while the sheet member is being pinched between the processing portion (46) and the sheet-placing surface (42a), the protuberance (47) is in contact with the sheet-placing surface on the other side closer to the other end in the axial direction than the sheet member is to the other end, and the other protuberance (48) abuts the sheet-placing surface (42a) on the side closer to the one end in the axial direction than the sheet member is to the one end,
a distance from the protuberance (47) to the center in the axial direction of the rotating roller (41) is longer than a distance from the processing portion (46) to the center in the axial direction and is longer than a distance from the other protuberance (48) to the center in the axial direction.

2. A processing apparatus according to claim 1, wherein
the processing apparatus further comprises a first blade as the processing portion, a second blade as the protuberance, and a third blade as the other protuberance, and
the sheet member is cut by being pinched between the sheet-placing surface (42a) and the first blade.

3. A processing apparatus according to claim 2, wherein
each of the second blade and the third blade is included on the peripheral surface in a manner of stretching around the whole perimeter along a rotating direction of the rotating roller (41),
a continuing direction of each of the second blade and the third blade is inclined at a certain angle with respect to the rotating direction.

4. A processing apparatus according to any one of claims 2 to 3, wherein
the rotating roller (41) is a first rotating roller that includes a first peripheral surface as the peripheral surface,
the sheet-placing portion (42) is a second rotating roller that includes a second peripheral surface as the sheet-placing surface and that is supported rotatably,
a motor (45) that rotates each of the first rotating roller and the second rotating roller is included individually on each of the rollers.

5. A processing apparatus according to claim 4, wherein
a circumferential velocity at which the first rotating roller is rotatable is different from a circumferential velocity at which the second rotating roller is rotatable.

6. A method for processing a sheet member used in manufacturing of an absorbent article, comprising:

preparing a rotating roller (41) whose both ends in an axial direction are supported; and
pinching the sheet member between a processing portion (46) and a sheet-placing surface (42a) while the rotating roller is rotating and the sheet member is placed on the sheet-placing surface of the sheet-placing portion, the processing portion (46) being included on a peripheral surface of the rotating roller (41) and being located at a side where one end of the rotating roller (41) is positioned with respect to a center of the rotating roller (41) in the axial direction of the rotating roller (41), wherein
when the sheet member is pinched between the processing portion (46) and the sheet-placing surface (42a) while the rotating roller (41) is rotating, a protuberance (47) abuts the sheet-placing surface (42a), the protuberance (47) being included on the peripheral surface and being located at an other side where an other end of the rotating roller (41) is positioned with respect to the center in the axial direction,
another protuberance (48) is disposed on the peripheral surface and is located closer to the side in the axial direction than the processing portion (46) is to the side,
while the sheet member is being pinched between the processing portion (46) and the sheet-placing surface (42a), the protuberance (47) is in contact with the sheet-placing surface on the other side closer to the other end in the axial direction than the sheet member is to the other end, and the other protuberance (48) abuts the sheet-placing surface on the side closer to the one end in the axial direction than the sheet member is to the one end, whereby
a distance from the protuberance (47) to the center in the axial direction of the rotating roller (41) is longer than a distance from the processing portion (46) to the center in the axial direction and is longer than a distance from the other protuberance (48) to the center in the axial direction.

**Patentansprüche**

1. Arbeitsmaschine zur Bearbeitung eines Bahnelements, das bei der Herstellung eines saugfähigen Artikels verwendet wird, umfassend:

eine rotierende Walze (41), die sich mit beiden ihrer Enden in eine axiale Richtung dreht, wobei sie gestützt werden;
einen Bahnenplatzierungsteil (42), der eine Bahnenplatzierungsfläche (42a) aufweist, auf der das Bahnelement platziert ist; und
einen Bearbeitungsteil (46) und einen Vorsprung (47), die an einer Umfangsfläche der rotierenden Walze eingeschlossen sind, wobei das Bahnelement bearbeitet wird, indem es zwischen der Bahnenplatzierungsfläche (42) und dem Bearbeitungsteil (46) eingeklemmt wird,
sich der Bearbeitungsteil (46) auf einer Seite befindet, an der ein Ende der rotierenden Walze (41) in Bezug auf einen Mittelpunkt der rotierenden Walze in der axialen Richtung der rotierenden Walze positioniert ist,
sich der Vorsprung (47) auf einer anderen Seite befindet, an der ein anderes Ende der rotierenden Walze in Bezug auf den Mittelpunkt in der axialen Richtung positioniert ist,
der Vorsprung (47) an der Bahnenplatzierungsfläche (42a) anliegt, wenn das Bahnelement zwischen dem Bearbeitungsteil (46) und der Bahnenplatzierungsfläche eingeklemmt ist, während sich die rotierende Walze (41) dreht,
**dadurch gekennzeichnet, dass**
die Arbeitsmaschine ferner einen anderen Vorsprung (48) umfasst, der an der Umfangsfläche angeordnet ist und sich näher an der Seite in der axialen Richtung befindet als der Bearbeitungsteil (46),
während das Bahnelement zwischen dem Bearbeitungsteil (46) und der Bahnenplatzierungsfläche (42a) eingeklemmt ist, der Vorsprung (47) mit der Bahnenplatzierungsfläche auf der anderen Seite näher an dem anderen Ende in der axialen Richtung als das Bahnelement in Kontakt steht, und der andere Vorsprung (48) an der Bahnenplatzierungsfläche (42a) auf der anderen Seite näher an dem einen Ende in der axialen Richtung als das Bahnelement anliegt, ein Abstand von dem Vorsprung (47) zum Mittelpunkt in der axialen Richtung der rotierenden Walze (41) länger ist als ein Abstand von dem Bearbeitungsteil (46) zu dem Mittelpunkt in der axialen Richtung und länger ist als ein Abstand von dem anderen Vorsprung (48) zum Mittelpunkt in der axialen Richtung.

2. Arbeitsmaschine nach Anspruch 1, wobei

die Arbeitsmaschine ferner eine erste Klinge als den Bearbeitungsteil, eine zweite Klinge als den Vorsprung und eine dritte Klinge als den anderen Vorsprung umfasst, und das Bahnelement geschnitten wird, indem es zwischen der Bahnenplatzierungsfläche (42a) und der ersten Klinge eingeklemmt wird.

3. Arbeitsmaschine nach Anspruch 2, wobei

jede der zweiten Klinge und der dritten Klinge an der Umfangsfläche auf eine Weise eingeschlossen ist, sodass sie sich um den gesamten Umfang entlang einer Drehrichtung der rotierenden Walze (41) ausdehnt, eine Verlaufsrichtung von jeder der zweiten Klinge und der dritten Klinge in Bezug auf die Drehrichtung in einem bestimmten Winkel geneigt ist.

4. Arbeitsmaschine nach einem der Ansprüche 2 bis 3, wobei

die rotierende Walze (41) eine erste rotierende Walze ist, die eine erste Umfangsfläche als die Umfangsfläche einschließt, der Bahnenplatzierungsteil (42) einer zweiten rotierenden Walze entspricht, die eine zweite Umfangsfläche als die Bahnenplatzierungsfläche einschließt und drehbar gestützt ist, ein Motor (45), der jede der ersten rotierenden Walze und der zweiten rotierenden Walze dreht, einzeln an jeder der Rollen eingeschlossen ist.

5. Arbeitsmaschine nach Anspruch 4, wobei sich eine Umfangsgeschwindigkeit, mit der die erste rotierende Walze gedreht werden kann, von einer Umfangsgeschwindigkeit unterscheidet, mit der die zweite rotierende Walze gedreht werden kann.

6. Verfahren zur Bearbeitung eines Bahnelements, das bei der Herstellung eines saugfähigen Artikels verwendet wird, umfassend:

Vorbereiten einer rotierenden Walze (41), von der beide Enden in einer axialen Richtung gestützt werden; und

Einklemmen des Bahnelements zwischen einem Bearbeitungsteil (46) und einer Bahnenplatzierungsfläche (42a), während sich die rotierende Walze dreht und das Bahnelement auf der Bahnenplatzierungsfläche des Bahnenplatzierungsteils platziert ist, wobei der Bearbeitungsteil (46) an einer Umfangsfläche der rotierenden Walze (41) eingeschlossen ist und sich auf einer Seite befindet, an der ein Ende der rotierenden Walze (41) in Bezug auf einen Mittelpunkt der rotierenden Walze (41) in der axialen Richtung der rotierenden Walze (41) positioniert ist, wobei, wenn das Bahnelement zwischen dem Bearbeitungsteil (46) und der Bahnenplatzierungsfläche (42a) eingeklemmt ist, während sich die rotierende Walze (41) dreht, ein Vorsprung (47) an der Bahnenplatzierungsfläche (42a) anliegt, wobei der Vorsprung (47) an der Umfangsfläche eingeschlossen ist und sich auf einer anderen Seite befindet, an der ein anderes Ende der rotierenden Walze (41) in Bezug auf den Mittelpunkt in der axialen Richtung positioniert ist, ein anderer Vorsprung (48) an der Umfangsfläche angeordnet ist und sich näher an der Seite in der axialen Richtung befindet als der Bearbeitungsteil (46), während das Bahnelement zwischen dem Bearbeitungsteil (46) und der Bahnenplatzierungsfläche (42a) eingeklemmt ist, der Vorsprung (47) mit der Bahnenplatzierungsfläche auf der anderen Seite näher an dem anderen Ende in der axialen Richtung als das Bahnelement in Kontakt steht, und der andere Vorsprung (48) an der Bahnenplatzierungsfläche auf der Seite näher an dem einen Ende in der axialen Richtung als das Bahnelement anliegt, wobei ein Abstand von dem Vorsprung (47) zum Mittelpunkt in der axialen Richtung der rotierenden Walze (41) länger ist als ein Abstand von dem Bearbeitungsteil (46) zu dem Mittelpunkt in der axialen Richtung und länger ist als ein Abstand von dem anderen Vorsprung (48) zum Mittelpunkt in der axialen Richtung.

**Revendications**

1. Appareil de traitement pour traiter un élément en feuille utilisé dans la fabrication d'un article absorbant, comprenant :

un rouleau rotatif (41) qui tourne avec ses deux extrémités dans une direction axiale étant supportée ; une partie de placement de feuille (42) ayant une surface de placement de feuille (42a) sur laquelle l'élément en feuille est placé ; et

une partie de traitement (46) et une protubérance (47) qui sont incluses sur une surface périphérique du rouleau rotatif, dans lequel l'élément en feuille est traité en étant pincé entre la surface de placement de feuille (42) et la partie de traitement (46),

la partie de traitement (46) est située sur un côté où une extrémité du rouleau rotatif (41) est positionnée par rapport à un centre du rouleau rotatif dans la direction axiale du rouleau rotatif,

la protubérance (47) est située d'un autre côté où une autre extrémité du rouleau rotatif est positionnée par rapport au centre dans la direction axiale,

la protubérance (47) bute contre la surface de placement de feuille (42a) lorsque l'élément en feuille est pincé entre la partie de traitement (46) et la surface de placement de feuille pendant que le rouleau rotatif (41) tourne, **caractérisé en ce que** l'appareil de traitement comprend en outre une autre protubérance (48) qui est disposée sur la surface périphérique et est située plus près du côté dans la direction axiale que l'est la partie de traitement (46) par rapport au côté,

tandis que l'élément en feuille est pincé entre la partie de traitement (46) et la surface de placement de feuille (42a), la protubérance (47) est en contact avec la surface de placement de feuille de l'autre côté plus près de l'autre extrémité dans la direction axiale que l'est l'élément en feuille par rapport à l'autre extrémité, et l'autre protubérance (48) bute contre la surface de placement de feuille (42a) sur le côté plus près de l'une extrémité dans la direction axiale que l'est l'élément en feuille par rapport à l'une extrémité,

une distance de la protubérance (47) au centre dans la direction axiale du rouleau rotatif (41) est plus longue qu'une distance de la partie de traitement (46) par rapport au centre dans la direction axiale et est plus longue qu'une distance de l'autre protubérance (48) au centre dans la direction axiale.

2. Appareil de traitement selon la revendication 1, dans lequel

l'appareil de traitement comprend en outre une première lame en tant que partie de traitement, une deuxième lame en tant que protubérance et une troisième lame en tant qu'autre protubérance, et l'élément en feuille est coupé en étant pincé entre la surface de placement de feuille (42a) et la première lame.

3. Appareil de traitement selon la revendication 2, dans lequel

chacune de la deuxième lame et de la troisième lame est incluse sur la surface périphérique de manière à s'étirer sur tout le périmètre le long d'une direction de rotation du rouleau rotatif (41),

une direction continue de chacune de la deuxième lame et de la troisième lame est inclinée d'un certain angle par rapport au sens de rotation.

4. Appareil de traitement selon l'une quelconque des revendications 2 à 3, dans lequel

le rouleau rotatif (41) est un premier rouleau rotatif qui inclut une première surface périphérique comme surface périphérique,

la partie de placement de feuille (42) est un second rouleau rotatif qui inclut une seconde surface périphérique en tant que surface de placement de feuille et qui est supporté en rotation,

un moteur (45) qui fait tourner chacun du premier rouleau rotatif et du second rouleau rotatif est inclus individuellement sur chacun des rouleaux.

5. Appareil de traitement selon la revendication 4, dans lequel

une vitesse circonférentielle à laquelle le premier rouleau rotatif peut tourner est différente d'une vitesse circonférentielle à laquelle le second rouleau rotatif peut tourner.

6. Procédé pour traiter un élément en feuille utilisé dans la fabrication d'un article absorbant, comprenant :

la préparation d'un rouleau rotatif (41) dont les deux extrémités dans une direction axiale sont supportées ; et

le pincement de l'élément en feuille entre une partie de traitement (46) et une surface de placement de feuille (42a) pendant que le rouleau rotatif tourne et l'élément en feuille est placé sur la surface de placement de feuille de la partie de placement de feuille, la partie de traitement (46) étant incluse sur une surface périphérique du rouleau rotatif (41) et étant située sur un côté où une extrémité du rouleau rotatif (41) est positionnée par rapport au centre du rouleau rotatif (41) dans la direction axiale du rouleau rotatif (41), dans lequel

lorsque l'élément en feuille est pincé entre la partie de traitement (46) et la surface de placement de feuille (42a) tandis que le rouleau rotatif (41) tourne, une protubérance (47) bute contre la surface de placement de feuille (42a), la protubérance (47) étant incluse sur la surface périphérique et étant située sur un autre côté où une autre extrémité du rouleau rotatif (41) est positionnée par rapport au centre dans la direction axiale,

une autre protubérance (48) est disposée sur la surface périphérique et est située plus près du côté dans la direction axiale que l'est la partie de traitement (46) par rapport au côté,
tandis que l'élément en feuille est pincé entre la partie de traitement (46) et la surface de placement de feuille (42a), la protubérance (47) est en contact avec la surface de placement de feuille de l'autre côté plus près de l'autre extrémité dans la direction axiale que l'est l'élément en feuille par rapport à l'autre extrémité, et l'autre protubérance (48) bute contre la surface de placement de feuille sur le côté plus près de l'une extrémité dans la direction axiale que l'est l'élément en feuille par rapport à l'une extrémité, moyennant quoi
une distance de la protubérance (47) au centre dans la direction axiale du rouleau rotatif (41) est plus longue qu'une distance de la partie de traitement (46) par rapport au centre dans la direction axiale et est plus longue qu'une distance de l'autre protubérance (48) au centre dans la direction axiale.

FIG. 1A

FIG. 1B

FIG. 1C

FIG. 2

FIG. 3

FIG. 4

FIG. 5A

21a

50

22a

TRANSPORTING
DIRECTION

10a

10a

FIG. 5B

20a

20b

22a

10a

TRANSPORTING
DIRECTION

20b

10a

20b

10a

20b

10a

20b

10a

FIG. 5C

FIG. 6

FIG. 7

23

FIG. 8

FIG. 9

AXIAL DIRECTION
(CD DIRECTION)

ONE END SIDE ◄───────────► OTHER END SIDE

41a

L3

L2

L1

L4 L4

48 46 47

41

FIRST
SUPPORT
POSITION

CENTER IN
AXIAL DIRECTION

SECOND
SUPPORT
POSITION

FIG. 10

AXIAL DIRECTION
(CD DIRECTION)

ONE END SIDE ◄───────────► OTHER END SIDE

48  20a  46        41a    22a    47

41

42

CENTER IN
AXIAL DIRECTION

FIG. 11

41a

M2

41

M1

20a  46

F

42

42a

FIRST
SUPPORT
POSITION

CENTER IN
AXIAL DIRECTION

SECOND
SUPPORT
POSITION

ONE END SIDE ◄───────────► OTHER END SIDE
AXIAL DIRECTION
(CD DIRECTION)

FIG. 12

CENTER IN
AXIAL DIRECTION 41a

ROTATING
DIRECTION

48 46

47

ONE END SIDE ← → OTHER END SIDE
AXIAL DIRECTION
(CD DIRECTION)
FIG. 13A

CENTER IN
AXIAL DIRECTION 41a

ROTATING
DIRECTION

46 47

ONE END SIDE ← → OTHER END SIDE
AXIAL DIRECTION
(CD DIRECTION)
FIG. 13B

CENTER IN
AXIAL DIRECTION

ROTATING
DIRECTION

49 49

46 47

49 49

ONE END SIDE ← → OTHER END SIDE
AXIAL DIRECTION
(CD DIRECTION)
FIG. 13C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11188699 A

- DE 102005060578 A1